# EUROPÄISCHE PATENTANMELDUNG

(11) **EP 0 865 792 A1**
(43) Veröffentlichungstag der Anmeldung: **23.09.1998**
(21) Anmeldenummer: 97810152.5
(22) Anmeldetag: 17.03.1997
(51) Int. Cl.: A61K 39/395, A61K 47/44, A61K 9/107

(54) **Pharmazeutische Zusammensetzung mit Immunglobulinen zur topischen Anwendung**

(71) Anmelder: ROTKREUZSTIFTUNG ZENTRALLABORATORIUM BLUTSPENDEDIENST SRK, CH-3000 Bern 22 (CH)
(72) Erfinder: Burek-Kozlowska, Anna, 3098 Schliern (CH); Bolli, Reinhard, 3076 Worb (CH); Moudry, Radmila, 3014 Bern (CH); Weder, Hans Georg, 6342 Baar (CH)
(74) Vertreter: BOVARD AG - Patentanwälte

(57) **Zusammenfassung**

Die vorliegende Erfindung betrifft eine neue vorteilhafte pharmazeutische Darreichungsform für Immunglobuline und Fragmente davon und ihre Herstellung. Die Darreichungsform ist als Nanoemulsion topisch applizierbar und enthält eine Kombination aus Phospholipiden, Triglyceriden und gegebenenfalls für topische Darreichungsformen geeignete wasserlösliche und/oder fettlösliche Hilfsstoffe.

## Beschreibung

Die vorliegende Erfindung betrifft eine pharmazeutische Zusammensetzung zur topischen Anwendung, die Immunglobuline als aktive Komponenten enthält und ein Verfahren zu ihrer Herstellung.

Immunglobulinpräparate, die aus gepooltem Plasma vieler Spender hergestellt werden, enthalten ein breites Spektrum an Antikörpern gegen Antigene von Viren, Pilzen und Bakterien sowie gegen deren Toxine. Solche kommerziell erhältlichen Präparate für den intravenösen oder intramuskulären Gebrauch vermögen Mikroorganismen und deren Toxine zu opsonieren und zu neutralisieren. Sie können aber auch auf Zellen des Immunsystems durch Stimulierung oder Hemmung der Zytokinproduktion Einfluss nehmen. Solche immunmodulatorische Eigenschaften dieser Präparate wurden in vitro und in vivo gezeigt (J.P. Andersson & U.G. Andersson, Immunology 71, 372-376 (1990); J.M. Dwyer, New England J. of Medicine 326, 107-116 (1992)). Deshalb können auch Autoimmunkrankheiten durch intravenös applizierte Immunglobulinpräparate erfolgreich behandelt werden. Durch ähnliche regulatorische Mechanismen können Immunglobuline auch Entzündungen hemmen. Ob eine direkte topische Anwendung von Immunglobulinen auf der Haut zur Behandlung von chronischen und akuten entzündlichen Hauterkrankungen (z.B. atopische Dermatitis, Psoriasis, HSV epitheliale Keratitis) geeignet ist, konnte bisher nicht abschliessend gezeigt werden (A. Burek-Kozlowska et al, Int. Arch. Immunology 104, 104-106 (1994); A.J. Kläger et al, Ophthalmologica 207, 78-81 (1993)).

Obschon die genaue Ursache bei der atopischen Dermatitis nicht bekannt ist, kann sie als typische Hauterkrankung gelten, bei der mikrobielle Infektionen mit allergischen und entzündlichen Merkmalen einhergehen, die wahrscheinlich auf falsch geleiteten Immunreaktionen beruhen. Die bakterielle Hautflora ist bei an dieser Krankheit leidenden Patienten stark verändert. Insbesondere ist ein starker Befall mit Staphylokokkus aureus auf der Hautoberfläche feststellbar. Eine Behandlung mit Antibiotika verbessert deshalb das klinische Erscheinungsbild der atopischen Dermatitis. Man nimmt an, dass die chronische atopische Dermatitis eine durch Allergene, die sich an die Langerhans-Zellen in der Epidermis binden, verursachte entzündliche Erkrankung der Haut darstellt. Es werden dabei verschiedene Zytokine (IL-4 und IL-5) freigesetzt, die wiederum Zellen (Mastzellen und Granulozyten) zur Freisetzung von entzündungsfördernden Mediatoren (z.B. TNF alfa, IL 10) stimulieren. Heute wird die atopische Dermatitis mit Kortikosteroidpräparaten behandelt, was aber bei längerer Behandlung zu Tachyphylaxie und Atrophie der Haut führt. Systemische Nebenwirkungen sind dabei nicht auszuschliessen. Aufgrund der oben erwähnten antibakteriellen und immunmodulatorischen Eigenschaften von Immunglobulinen ist eine nebenwirkungsfreie Behandlung der atopischen Dermatitis mit Immunglobulinen erfolgversprechend, sofern der Wirkstoff in die entsprechenden Hautschichten gelangen kann.

Rein wässerige Formulierungen von Immunglobulinen sind schlecht geeignet, um den Durchtritt dieser hydrophilen und zum Teil hochmolekularen Strukturen durch das Stratum corneum und in die relevanten Bereiche der Haut zu fördern.

Es ist demzufolge Aufgabe der vorliegenden Erfindung, eine Darreichungsform zur Verfügung zu stellen, welche eine topische Applikation von Immunglobulinen erlaubt.

In der Europäischen Patentschrift Nr. 406 162 (Weder et al.) und in der US-Patentschrift Nr. 5,152,923 (Weder et al.) ist ein Verfahren beschrieben, bei dem mittels einem Hochdruckhomogenisator Nanoemulsionen mit einer mittleren Teilchengrösse < 200 nm, vorzugsweise <100 nm (Mittelwert Gauss-Analyse-Anzahlsverteilung) von dispergierten Lipiden (Phospholipid der Formel I und Triglycerid der Formel II, siehe unten) hergestellt werden, ebenfalls wurde die Verwendung dieser Nanoemulsionen für die Herstellung von pharmazeutischen Darreichungsformen mit niedermolekularen Wirkstoffen vorgeschlagen.

Es wurde nun gefunden, dass derartige Nanopartikel auch als Träger für Proteine und Peptide, insbesondere Immunglobuline oder Fragmente davon, geeignet sind, wobei ihre Penetration durch das Stratum Corneum erleichtert wird und der Wirkstoff den Wirkungsort erreichen kann.

Gegenstand der vorliegenden Erfindung ist eine pharmazeutische Zusammensetzungen enthaltend:
a) mindestens ein Immunglobulin, der Klassen A, G, M, D, E, oder eine Subklasse oder ein Fragment davon
b) mindestens ein synthetisches oder natürliches Phospholipid der Formel: 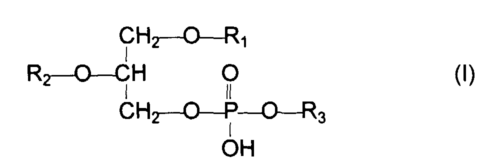
   worin R₁ C₁₀₋₂₀-Acyl ist, R₂ Wasserstoff oder C₁₀₋₂₀-Acyl ist, R₃ Wasserstoff, oder gegebenenfalls durch Amino, alkylsubstituiertes Amino, Hydroxy oder Carboxy substituiertes C₁₋₅-Alkyl bedeutet oder pharmazeutisch annehmbare Salze davon;
   R₃ kann vorzugsweise 2-Trimethylammmonium-1-ethyl, 2-Amino-1-ethyl, C₁₋₄-Alkyl, durch Carboxy substituiertes C₁₋₅-Alkyl, durch Hydroxy substituiertes C₂₋₅-Alkyl, durch Carboxy und Hydroxy substituiertes C₂₋₅-Alkyl oder durch Carboxy und Amino substituiert C₂₋₅-Alkyl bedeuten.
c) ein synthetisches oder natürliches Triglycerid der Formel: 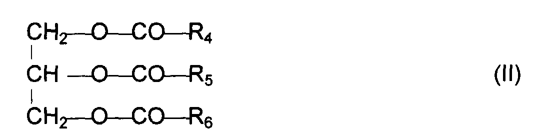
   worin R₄, R₅ und R₆ C₈₋₂₀-Acyl bedeuten;
d) Wasser als Trägerflüssigkeit in der für die topische Applikation erforderlichen Reinheit;
   sowie folgende fakultative Komponente:
e) für die pharmazeutische Darreichungsform geeignete wasserlösliche oder fettlösliche Hilfsstoffe.

Die weiter vorn und im folgenden genannten Begriffe werden im Rahmen der Beschreibung der vorliegenden Erfindung wie folgt definiert:

### Komponente a):

Das Immunglobulin kann natürlichen Ursprungs (tierisch oder human) sein oder durch Hybridoma- oder Rekombinationstechnik hergestellt, mono-, oligo- oder polyklonal, chimärisiert oder humanisiert sein. Es kann aber auch aus F(ab)-, F(ab)₂-, Fc- oder synthetischen (z.B. Einzelstrang-Fv) Immunglobulin-Fragmenten bestehen. Das Immunglobulin und Fragmente davon können modifiziert vorliegen, z.B. am gleichen Molekül zwei Spezifitäten aufweisen (bispezifisch), oder modifiziert mit Lipidanker oder Membrandomänen versehen sein.

### Komponente b):

Die Nomenklatur der Phospholipide (I) und die Bezifferung der C-Atome erfolgt anhand der in Eur.J.of Biochem. 79, 11-21 (1977) "Nomenclature of Lipids" von der IUPAC-IUB Commission on Biochemical Nomenclature (CBN) gegebenen Empfehlungen (sn-Nomenklatur, stereospecific numbering).

R₁ und R₂ mit den Bedeutungen C₁₀₋₂₀-Acyl sind vorzugsweise geradkettiges C₁₀₋₂₀-Alkanoyl mit einer geraden Anzahl an C-Atomen und geradkettiges C₁₀₋₂₀-Alkenoyl mit einer oder mehreren Doppelbindungen und einer geraden Anzahl an C-Atomen.

Geradkettiges C₁₀₋₂₀-Alkanoyl R₁ und R₂ mit einer geraden Anzahl von C-Atomen sind beispielsweise n-Dodecanoyl, n-Tetradecanoyl, n-Hexadecanoyl oder n-Octadecanoyl.

Geradkettiges C₁₀₋₂₀-Alkenoyl R₁ und R₂ mit einer Doppelbindung und einer geraden Anzahl an C-Atomen sind beispielsweise 6-cis- oder 6-trans-, 9-cis- oder 9-trans-Dodecenoyl, -Tetradecenoyl, -Hexadecenoyl, -Octadecenoyl oder -Icosenoyl, insbesondere 9-cis-Octadecenoyl (Oleoyl).

Ein Phospholipid (I), worin R₃ 2-Trimethylamino-1-ethyl bedeutet, wird mit dem Trivialnamen Lecithin und ein Phospholipid (I), worin R₃ 2-Amino-1-ethyl bedeutet, mit dem Trivialnamen Kephalin bezeichnet. Geeignet sind beispielsweise natürlich vorkommendes Kephalin oder Lecithin, z.B. Kephalin oder Lecithin aus Sojabohnen oder Hühnerei mit verschiedenen oder identischen Acylgruppen R₁ und R₂, oder Mischungen davon.

Das Phospholipid (I) kann aber auch synthetischen Ursprungs sein. Unter dem Begriff Phospholipid definiert man Phospholipide, welche bezüglich R₁ und R₂ eine einheitliche Zusammensetzung haben. Solche synthetischen Phospholipide sind vorzugsweise die weiter vorn definierten Lecithine und Kephaline, deren Acylgruppen R₁ und R₂ eine definierte Struktur haben und von einer definierten Fettsäure mit einem Reinheitsgrad höher als ca. 95% abgeleitet sind. R₁ und R₂ können gleich oder verschieden und ungesättigt oder gesättigt sein. Bevorzugt ist R₁ gesättigt, z.B. n-Hexadecanoyl, und R₂ ungesättigt, z.B. 9-cis-Octadecenoyl (=Oleyl).

Der Begriff "natürlich vorkommendes" Phospholipid (I) definiert Phospholipide, welche bezüglich R₁ und R₂ keine einheitliche Zusammensetzung haben. Solche natürlichen Phospholipide sind ebenfalls Lecithine und Kephaline, deren Acylgruppen R₁ und R₂ strukturell undefinierbar und von natürlich vorkommenden Fettsäuregemischen abgeleitet sind.

Die Forderung "im wesentlichen reines" Phospholipid (I) definiert einen Reinheitsgrad von mehr als 90% (Gew.), vorzugsweise mehr als 95%, des Phospholipids (I), welcher anhand geeigneter Bestimmungsmethoden, z.B. papierchromatographisch, mit Dünnschichtchromatographie, mit HPLC oder enzymatischem Farbtest, nachweisbar ist.

In einem Phospholipid (I) ist R₃ mit der Bedeutung C₁₋₄-Alkyl beispielsweise Methyl oder Ethyl. Die Bedeutung Methyl ist bevorzugt.

R₃ mit den Bedeutungen C₁₋₅-Alkyl substituiert durch Carboxy, C₂₋₅-Alkyl substituiert durch Hydroxy oder C₂₋₅-Alkyl substituiert durch Carboxy oder Hydroxy sind beispielsweise 2-Hydroxyethyl, 2,3-Dihydroxy-n-propyl, Carboxymethyl, 1- oder 2-Carboxyethyl, Dicarboxymethyl, 2-Carboxy-2-hydroxyethyl oder 3-Carboxy-2,3-dihydroxy-n-propyl.

R₃ mit der Bedeutung C₂₋₅-Alkyl substituiert durch Carboxy und Amino ist z.B. 3-Amino-3-carboxy-n-propyl oder 2-Amino-2-carboxy-n-propyl, vorzugsweise 2-Amino-2-carboxyethyl. Phospholipide (II) mit diesen Gruppen können in Salzform, z.B. als Natrium- oder Kaliumsalz, vorliegen.

Für die Acylreste in den Phospholipiden (I) sowie in den Triglyceriden (II) sind auch die in Klammern angegebenen Bezeichnungen gebräuchlich:

9-cis-Dodecenoyl (Lauroleoyl), 9-cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 11-cis-Octadecenoyl (Vaccenoyl), 9-cis-Icosenoyl (Gadoleoyl), n-Dodecanoyl (Lauroyl), n-Tetradecanoyl (Myristoyl), n-Hexadecanoyl (Palmitoyl), n-Octadecanoyl (Stearoyl), n-Icosanoyl (Arachidoyl).

Ein Salz des Phospholipids (I) ist pharmazeutisch annehmbar. Salze definieren sich durch die Existenz von salzbildenden Gruppen im Substituenten R₃, sowie durch die freie Hydroxygruppe am Phosphor. Möglich ist ebenfalls die Bildung von inneren Salzen. Bevorzugt sind Alkalimetallsalze, insbesondere Natriumsalze.

In einer besonders bevorzugten Ausführungsform verwendet man gereinigtes Lecithin aus Sojabohnen.

### Komponente c):

In einem als Komponente c) verwendeten Triglycerid der Formel (II) bedeuten R₄, R₅ und R₆ geradkettiges C₈₋₂₀-Acyl mit gerader Anzahl an C-Atomen, insbesondere n-Octanoyl, n-Dodecanoyl, n-Tetradecanoyl, n-Hexadecanoyl, n-Octadecanoyl, 9-cis-Dodecanoyl, 9-cis-Tetradecenoyl, 9-cis-Hecadecenoyl, 9-cis-Octadecenoyl oder 9-cis-Icosenoyl. Die Bedeutungen von R₄, R₅ und R₆ können identisch oder verschieden sein, wobei die individuellen Gruppen R₄, R₅ und R₆ selbst strukturell einheitlich definiert sind. Dies ist für synthetische oder halbsynthetische Triglyceride charakteristisch. R₄, R₅ und R₆ können aber auch aus verschiedenen Acylgruppen unterschiedlicher Struktur bestehen. Dies ist für Triglyceride natürlichen Ursprungs charakteristisch.

Ein Triglycerid der Formel (II) ist ein halbsynthetisches oder synthetisches, im wesentlichen reines Triglycerid oder pharmazeutisch gebräuchliches Triglycerid natürlichen Ursprungs. Bevorzugt ist ein Triglycerid natürlichen Ursprungs, z.B. Erdnuss-, Sesam-, Sonnenblumen-, Oliven-, Maiskeim-, Soja-, Rizinus-, Baumwollsamen-, Raps-, Distel-, Traubenkern-, Fisch- oder Kokosöl. In einer besonders bevorzugten Ausführungform der Erfindung verwendet man ein mit dem Begriff Neutralöl definiertes Triglycerid mit verschiedenen Acylgruppen unterschiedlicher Struktur, z.B. ein Triglycerid der fraktionierten Kokosfettsäuren C₈-C₁₀ vom Typ Miglyol®, z.B. MIGLYOL 812.

### Trägerfüssigkeit:

Die Trägerflüssigkeit Wasser ist in der für topische (dermale) Applikationen erforderlichen Reinheit nach den Vorschriften der nationalen Pharamakopöen, wie U.S. Pharmakopöe (USP) oder Deutsches Arzneibuch (DAB), vorgeschriebenen Reinheit keimfrei.

### Hilfsstoffe:

Für topische (dermale) Arzneiformen sind ohne Einschränkungen die für Formulierungen wie o/w-, w/o-, w/o/w-Emulsionen, Gele, Salben, Lotionen, Sprays etc. bekannten und zulässigen wasserlöslichen und/oder fettlöslichen Hilfsstoffe, einschliesslich Konservierungsmittel und Antioxidantien, geeignet.

Eine bevorzugte Ausführungsform betrifft eine pharmazeutische Zusammensetzung enthaltend:
a) ein Immunglobulin, der Klassen A, G, M, D, E, oder deren Subklassen zugehörend, Gemische und Fragmente davon;
b) gereinigtes Lecithin aus Sojabohnen;
c) ein Triglycerid aus der Gruppe der Neutralöle;
d) Wasser als Trägerflüssigkeit; und
e) für topische (dermale) Arzneiformen geeignete zulässige wasserlösliche und/oder fettlösliche Hilfsstoffe.

Ebenfalls Gegenstand der vorliegenden Erfindung ist das an sich bekannte Verfahren zur Herstellung der pharmazeutischen Zusammensetzung, welches dadurch gekennzeichnet ist, dass man eine Liposomensuspension enthaltend das Phospholipid der Formel I und gegebenenfalls wasserlösliche Hilfsstoffe herstellt, diese wässerige Phase mit dem Triglycerid der Formel II vermischt und dispergiert und anschliessend den Bedingungen der Hochdruckhomogenisation unterwirft, und die dadurch erhaltene Nanoemulsion den folgenden Nachoperationen unterzieht:

Zusatz einer weiteren Menge Wasser als Trägerflüssigkeit sowie gegebenenfalls weiterer wasserlöslicher Hilfsstoffe, welche für topische Arzneiformen (Dermatika) geeignet sind, Filtration insbesondere 0.2 µm-Sterilfiltration der Nanoemulsion.

Die sterile und pH-stabilisierte Immunglobulin-Lösung wird mit der sterilen Nanoemulsion vermischt und bei Raumtemperatur unter Rühren (Magnetrührwerk) inkubiert. Die Immunglobulin-Nanoemulsion wird folgenden Nachoperationen unterzogen:

Zusatz einer weiteren Menge Wasser als Trägerflüssigkeit sowie gegebenenfalls weiterer wasserlöslicher Hilfsstoffe, welche für topische Arzneiformen (Dermatika) geeignet sind, Filtration insbesondere 0.2 µm-Sterilfiltration der Immunglobulin-Nanoemulsion und gegebenenfalls Einarbeitung dieser Immunglobulin-Nanoemulsion unter aseptischen Bedingungen in topische (dermale) Formulierungen wie o/w-, w/o-, w/o/w-Emulsionen, Gele, Salben, Lotionen, Sprays etc.

Unter den Bedingungen der Hochdruckhomogenisation bildet sich eine Dispersion mit physikochemischen Eigenschaften, die mit dem Begriff Nanoemulsion definierbar sind. Eine Nanoemulsion lässt sich als kolloid-hochdisperses Zweiphasensystem auffassen. Aufgrund von Laser-Lichtstreumessungen und Aufnahmen im Elektronenmikroskop sind die in der Dispersion vorhandenen amphiphilen Partikel von anderen Gebilden wie Flüssigkristallen, Mizellen, Umkehrmizellen oder Liposomen unterscheidbar. Für die statistische Mehrzahl von mehr als 95 % ist eine mittlere Partikelgrösse kleiner als 150 nm (Gauss-Analyse Anzahlsverteilung, siehe unten) charakteristisch. Die Immunglobulin-Moleküle interagieren mit der Monolayermembran der Nanopartikel mit definierter Affinität, wodurch die mittlere Nanopartikelgrösse signifikant zunimmt.

Zur Charakterisierung der nach dem oben beschriebenen Verfahren erhaltenen Immunglobulin-Nanoemulsion sind an sich bekannte Methoden geeignet, z.B. optische Beurteilung nach Verdünnung mit Wasser: schwache bis starke Opaleszenz des Präparats ist leicht erkennbar (Hinweis auf durchschnittliche Partikelgrösse kleiner 150 nm); Gelpermeationschromatographie; Laser-Lichtstreumessung (Bestimmung der Partikelgrösse und Homogenität: Anzahl-, Volumen- und Intensitätsverteilung in Form von Gauss-Analysen); Elektronenmikroskopie (Gefrierbruch- und Negativkontrasttechnik).

Die weiter vorn beschriebenen pharmazeutischen Zusammensetzungen sind als dermal bzw. topisch verabreichte Arzneimittel zur Behandlung von Erkrankungen vorzugsweise Entzündungen und Autoimmunerkrankungen der Haut und Schleimhaut (z.B. atopische Dermatitis, Psoriasis, Akne etc.) therapeutisch anwendbar.

Die folgenden Beispiele dienen der näheren Erläuterung der vorliegenden Erfindung:

### Beispiel 1:

### a) Rezeptur für eine IgG 5 %-Nanopartikel-Dosiersprayformulierung. Prozentangaben in (w/w).

- 25.0 %: IgG-Lösung 20 %, pH 4 bis 6
- 1.80 %: Lecithin aus Sojabohnenöl (LIPOID S100)
- 5.45 %: Neutralöl Miglyol 812
- 67.75 %: Aqua purificata

### b) Ansatzmenge für 5 kg Ansatz:

- 1250.0g: IgG-Lösung 20%, pH 4 bis 6
- 90.0 g: Lecithin aus Sojabohnenöl (LIPOID S100)
- 272.5 g: Neutralöl Miglyol 812
- 3387.5 g: Aqua purificata

### Herstellung einer IgG-Lösung 20 %:

Roh-IgG-Paste, die aus vereintem Blutplasma vieler Spender durch Alkohol-Fraktionierung erhalten wurde, wird in sterilem Wasser aufgelöst und bei 4 °C filtriert. Die Lösung wird mit Salzsäure auf pH 4 eingestellt und mit einer minimalen Menge Pepsin bei pH 4 und 37 °C inkubiert und wieder neutralisiert. Das Produkt wird, um letzte Mengen an Alkohol zu entfernen, diafiltriert, auf pH 5.5 eingestellt und durch Ultrafiltration auf 20 % Proteingehalt aufkonzentriert.

Das Produkt kann auch, wie im Schweizer Patent No. 684 164 beschrieben, hergestellt werden.

Das Lecithin aus Sojabohnenöl (Lipoid S100) wird vorgelegt und mit Aqua purificata vermischt. Man dispergiert mit einem Dispergator vom Typ Polytron PT 3000 (pH 6-8, Raumtemperatur, 10'000 Upm, Endtemperatur 25-28 °C, 5 min).

Nach Vermischen des Neutralöls Miglyol 812 mit der wässerigen Liposomensuspension dispergiert man während 5 min bei 10'000 Upm (pH 6-8, Raumtemperatur, Endtemperatur 25-28 °C) und unterwirft das Gemisch anschliessend der Hochdruckhomogenisation (Hochdruckhomogenisator Rannie Typ LAB/HP 12.51 H, 700 bar, 18 min, entspricht 10 Zyklen). Als Verfahrenstemperatur wählt man den Bereich 34-38 °C. Die erhaltene Nanoemulsion wird anschliessend über 0.2 µm Gelman-Filter sterilfiltriert.

Die sterile Nanoemulsion wird vorgelegt und mit der sterilen IgG-Lösung 20 % unter aseptischen Bedingungen vermischt (Raumtemperatur, Magnetrührwerk, 60 min). Die erhaltene IgG-Nanoemulsion wird anschliessend über 0.2 µm Gelman-Filter sterilfiltriert.

### Beispiel 2:

### a) Rezeptur für eine IgG 5 %-Lipogel-Dosierspenderformulierung. Prozentangaben in (w/w).

- 25.00 %: IgG-Lösung 20 %, pH 4 bis 6
- 1.80 %: Lecithin aus Sojabohnenöl (LIPOID S100)
- 5.45%: Neutralöl Miglyol 812
- 2.30 %: Gelbildner Liporamnosan® (Hydroxyethylcellulose)
- 65.45 %: Aqua purificata

### b) Ansatzmenge für 5 kg Ansatz:

- 1250.0g: IgG-Lösung 20%, pH 4 bis 6
- 90.0 g: Lecithin aus Sojabohnenöl (LIPOID S100)
- 272.5g: Neutralöl Miglyol 812
- 115.0 g: Gelbildner Liporamnosan® (Hydroxyethylcellulose)
- 3272.5 g: Aqua purificata

Die Herstellung erfolgt analog Beispiel 1. Die IgG-Nanoemulsion wird im Stephan UMC12 vorgelegt und der Gelbildner unter aseptischen Bedingungen zugegeben und vermischt (25 °C, Vakuum, 300 Upm, 15 min). Anschliessend wird das erhaltene Filtrat während 30 min im Vakuum stehen gelassen.

### Beispiel 3:

a) Herstellung der Prüfprodukte und
b) Messung der Immunglobulinpenetration in die Haut der haarlosen Ratte.
   a) Eine IgG 5 % Nanopartikel-Formulierung (Nanoemulsion) wird, wie unter Beispiel 1 beschrieben, hergestellt.
      Die IgG-Lösung wird durch Auflösen von Sandoglobulin® gemäss dem Beschrieb im Beipackzettel mit physiologischer Kochsalzlösung und Verdünnen mit sterilem Wasser auf einen Proteingehalt von 5 % hergestellt.
   b) Die Messung wird in der Diffusionszelle nach Franz (T.J. Franz, J. Invest. Dermatol. 64, 190-195 (1975)) durchgeführt. Ein Hautstück einer haarlosen Ratte (CRI:CD^{R} hr/hr, Charles River) wird in die Zelle eingespannt und auf der Unterseite mit 0.9 % NaCl bespühlt. 120 µl der Prüfprodukte werden aufgetragen und während 24 h bei 37 °C inkubiert. Die Hornhaut wird anschliessend durch mehrmaliges Strippen mit Klebeband entfernt, die restliche Haut mit Kollagenase behandelt und homogenisiert. Die im Homogenat angereicherte Menge des Immunglobulins wird im Immunoblott quantifiziert. Die Ergebnisse dieser Untersuchungen sind in nachstehender Tabelle zusammengefasst.

**Tabelle**

| Penetration von IgG durch das Stratum Corneum: | | | |
|---|---|---|---|
| Prüfprodukte mit 5 % Proteingehalt | Anzahl Experimente | Menge des durch das Stratum Corneum diffundierten Immunglobulins | |
| | | µg IgG / cm² Haut * | % |
| wässrige IgG-Lösung | 4 | 5.7 ± 1.4 | 1.67 |
| IgG-Nanopartikel-Formulierung | 7 | 8.3 ± 2.05 | 2.45 |

| | | | |
|---|---|---|---|
| * Mittelwerte ± s.d. | | | |

## Patentansprüche

1. Pharmazeutische Zusammensetzung für die topische Applikation von Immunglobulinen, enthaltend
a) mindestens ein Immunglobulin der Klassen A, G, M, D, E, einer Subklasse davon, ein Fragment eines solchen Immunglobulins oder ein chemisch modifizierter Vertreter eines solchen Immunglobulins;
b) ein synthetisches oder natürliches Phospholipid der Formel: 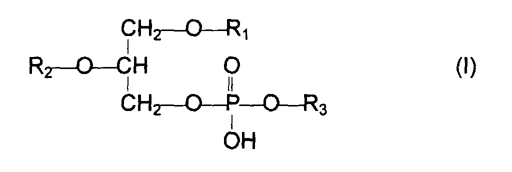
worin R₁ C₁₀₋₂₀-Acyl ist, R₂ Wasserstoff oder C₁₀₋₂₀-Acyl ist, R₃ Wasserstoff, oder gegebenenfalls durch Amino, alkylsubstituiertes Amino, Hydroxy oder Carboxy substituiertes C₁₋₅-Alkyl bedeutet oder pharmazeutisch annehmbare Salze davon;
c) ein synthetisches oder natürliches Triglycerid der Formel: 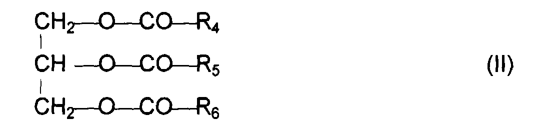
worin R₄, R₅ und R₆ unabhängig voneinander C₈₋₂₀Acyl bedeuten;
in wässeriger Emulsion mit einer mittleren Partikelgrösse von 50 bis 300 nm.

2. Pharmazeutische Zusammensetzung gemäss Anspruch 1, dadurch gekennzeichnet, dass R₁ und R₂ unabhängig voneinander geradkettiges C₁₀₋₂₀-Alkanoyl mit einer geraden Anzahl an C-Atomen, beispielsweise n-Dodecanoyl, n-Tetradecanoyl, n-Hexadecanoyl oder n-Octadecanoyl, oder geradkettiges C₁₀₋₂₀-Alkenoyl mit einer oder mehreren Doppelbindungen und einer geraden Anzahl an C-Atomen, beispielsweise 6-cis-oder 6-trans-, 9-cis- oder 9-trans-Dodecenoyl, -Tetradecenoyl, -Hexadecenoyl, -Octadecenoyl oder -Icosenoyl, insbesondere 9-cis-Octadecenoyl bedeutet.

3. Pharmazeutische Zusammensetzung gemäss Anspruch 1 oder 2, dadurch gekennzeichnet, dass R₃ Methyl, Ethyl, 2-Hydroxyethyl, 2,3-Dihydroxy-n-propyl, Carboxymethyl, 1-Carboxyethyl, 2-Carboxyethyl, Dicarboxymethyl, 2-Carboxy-2-hydroxyethyl, 3-Carboxy-2,3-dihydroxy-n-propyl, 3-Amino-3-carboxy-n-propyl, 2-Amino-2-carboxy-n-propyl oder 2-Amino-2-carboxyethyl bedeutet.

4. Pharmazeutische Zusammensetzung gemäss einem der Ansprüche 1 bis 3, dadurch gekennzeichnet, dass R₄, R₅ und R₆ unabhängig voneinander n-Octanoyl, n-Dodecanoyl, n-Tetradecanoyl, n-Hexadecanoyl, n-Octadecanoyl, 9-cis-Dodecanoyl, 9-cis-Tetradecenoyl, 9-cis-Hecadecenoyl, 9-cis-Octadecenoyl oder 9-cis-Icosenoyl bedeuten.

5. Pharmazeutische Zusammensetzung gemäss Anspruch 1 enthaltend:
a) ein Immunglobulin das im wesentlichen der Klasse G oder A zugehört oder Fragmente davon;
b) gereinigtes Lecithin aus Sojabohnen;
c) ein natürliches Triglycerid aus der Gruppe der Neutralöle;
d) für topische Arzneiformen annehmbare wasserlösliche und/oder fettlösliche Hilfsstoffe.

6. Pharmazeutische Zusammensetzung nach einem der Ansprüche 1 bis 5, dadurch gekennzeichnet, dass das Immunglobulin der Klassen A, G, M, D, E oder deren Subklassen, ein poly-, oligo- oder monoklonales Immunglobulin natürlicher, synthetischer oder rekombinanter Herkunft ist, und humaner, tierischer, chimärischer oder humanisierter Bauart ist.

7. Pharmazeutische Zusammensetzung nach einem der Ansprüche 1 bis 6, dadurch gekennzeichnet, dass als aktive Komponenente Fragmente von Immunglobulinen der Klassen A, G, M, D, E oder deren Subklassen ausgewählt aus der Gruppe bestehend aus F(ab)-, F(ab)₂- oder Fc-Immunglobulin-Fragmenten oder synthetische Immunglobulin-Fragmente, z.B. Einzelstrang-Fv-Fragmente verwendet werden.

8. Pharmazeutische Zusammensetzung nach einem der Ansprüche 1 bis 4, dadurch gekennzeichnet, dass das Immunglobulin chemisch derart modifiziert ist, dass es mehrere Spezifitäten pro Molekül, einen Lipidanker oder Mebrandomänenen aufweist;

9. Pharmazeutische Zusammensetzung nach einem der Ansprüche 1 bis 8, dadurch gekennzeichnet, dass sie die Form einer Nanoemulsion mit mittleren Partkelgrössen von ≤ 200 nm und vorzugsweise von ≤ 100 nm aufweist.

10. Pharmazeutische Zusammensetzung nach einem der Ansprüche 1 bis 9 zur therapeutischen Behandlung von Erkrankungen der Haut und Schleimhaut, wie von Entzündungen und Autoimmunerkrankungen der Haut und Schleimhaut.

11. Verfahren zur Herstellung einer pharmazeutischen Zusammensetzung gemäss einem der Ansprüche 1 bis 10 für die topische Applikation eines Immunglobulins, gekennzeichnet durch folgende Schritte:
a) Herstellung einer wässerigen Liposomensuspension aus einem Phospholipid der Formel I 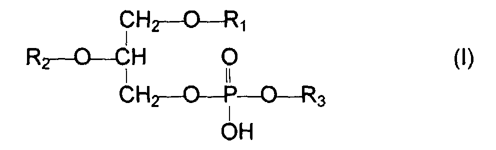
worin R₁ C₁₀₋₂₀-Acyl ist, R₂ Wasserstoff oder C₁₀₋₂₀-Acyl ist, R₃ Wasserstoff, oder gegebenenfalls durch Amino, alkylsubstituiertes Amino, Hydroxy oder Carboxy substituiertes C₁₋₅-Alkyl bedeutet oder aus einem pharmazeutisch annehmbaren Salz davon;
b) Zugabe eines synthetischen oder natürlichen Triglycerides der Formel II: 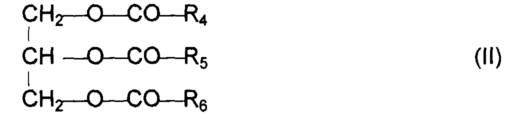
worin R₄, R₅ und R₆ unabhängig voneinander C₈₋₂₀-Acyl bedeuten, zu der im Schritt a) erhaltenen Liposomensuspension unter Mischen und Dispergieren
c) Homogenisation der im Schritt b) erhaltenen Dispersion, wobei eine Emulsion erhalten wird, welche anschliessend mit Wasser verdünnt und sterilfiltriert wird,
d) Zugabe einer Lösung enthaltend mindestens ein Immunglobulin der Klassen A, G, M, D, E, einer Subklasse davon, ein Fragment eines solches Immunglobulins oder ein chemisch modifzierter Vertreter eines solchen Immunglobulins zu der in Schritt c) erhaltenen sterilfiltrierten Dispersion und
e) Inkubation der in Schritt d) erhaltenen Suspension bei 20 ± 10 °C und Überführung in eine Nanoemulsion mit mittleren Partikelgrössen von 50 bis 300 nm.

12. Verfahren nach Anspruch 11, dadurch gekennzeichnet, dass nach der Inkubation mit einer weiteren Menge Wasser als Trägerflüssigkeit verdünnt und mit einem 0.2 µm-Sterilfilter filtriert wird, derart, dass eine Nanoemulsion mit einer mittleren Partikelgrösse von ≤ 200 nm und vorzugsweise von ≤ 100 nm gebildet wird.

13. Verfahren nach Anspruch 11 oder 12, dadurch gekennzeichnet, dass die erhaltene Suspension unter Zugabe von weiteren wasserlöslichen Hilfsstoffen, in topische Arzneiformen, wie Dermatika übergeführt wird.

14. Verfahren nach Anspruch 13, dadurch gekennzeichnet, dass die erhaltene Zusammensetzung in Form einer Nanoemulsion in dermale Formulierungen in Form von o/w-, w/o-, w/o/w-Emulsionen, Gelen, Salben, Lotionen, Sprays u.a. übergeführt werden.
